# EUROPEAN PATENT APPLICATION

(11) **EP 4 512 795 A1**
(43) Date of publication of application: **26.02.2025**
(21) Application number: 23193313.6
(22) Date of filing: 24.08.2023
(51) Int. Cl.: C07C 67/54, C07C 67/333, C07C 67/52, C07C 69/54

(54) **A METHOD AND PLANT FOR RECYCLING POLYMETHYL METHACRYLATE**

(71) Applicant: Sulzer Management AG, 8401 Winterthur (CH)
(72) Inventor: STEPANSKI, Manfred, 9470 Buchs (CH)
(74) Representative: IPS Irsch AG

(57) **Abstract**

The present invention relates to a method for preparing a purified methyl methacrylate composition from a crude composition containing methyl methacrylate comprising the steps of:
a) providing as crude composition a pyrolyzed composition obtained by pyrolysis of a polymethyl methacrylate composition,
b) subjecting the pyrolyzed composition to at least one distillation step so as to obtain a methyl methacrylate enriched composition and
c) then subjecting the methyl methacrylate enriched composition to at least one melt crystallization step so as to obtain the purified methyl methacrylate composition.

## Description

The present invention relates to a method and a plant for preparing a purified methyl methacrylate composition from a crude composition containing methyl methacrylate, such as in particular from a crude composition being prepared by pyrolysis of a composition containing recycled polymethyl methacrylate. More specifically, the present invention relates to a method and a plant, which is able to separate light, heavy and close boiling components including components that influence the color from methyl methacrylate so as to obtain a methyl methacrylate of a high quality being in particular suitable to produce high quality polymethyl methacrylate or optical grade polymethyl methacrylate.

Polymethyl methacrylate, which is also known as acrylic glass as well as by the trade names and brands Crylux, Alfaplas, Plexiglas, Acrylite, Lucite, and Perspex, among several others, is one of the most widely used plastics worldwide, among others because it is a transparent thermoplastic being excellently moldable to the desired form, such as by injection molding. Polymethyl methacrylate is for instance used as material of optical lenses, spectacle lenses, glazing, lamps, dental prostheses, medical protheses, covers and lenses of exterior lights of automobiles, sanitary parts, such as lavatories, and the like.

The recycling of polymethyl methacrylate waste becomes more and more important. Firstly, polymethyl methacrylate is a homopolymer of methyl methacrylate, which is typically produced by starting materials deriving from raw oil or from hydrocarbon streams deriving from raw oil, respectively. Therefore, is not only from an economical viewpoint, but in particular also from an ecological viewpoint advantageous to recover the raw material methyl methacrylate from polymethyl methacrylate waste with the possibility for the reproduction of the polymer polymethyl methacrylate. Furthermore, the disposal of polymethyl methacrylate is challenging and expensive on account of the presence of contaminants of different nature within the polymethyl methacrylate waste.

Usually, the recycling of polymethyl methacrylate waste bases on the thermal decomposition of polymethyl methacrylate in a pyrolysis reactor to methyl methacrylate. However, the pyrolysis of polymethyl methacrylate leads to a plurality of side-products, which have to be separated from the methyl methacrylate, before the methyl methacrylate may be used for instance as monomer for the production of new of polymethyl methacrylate compared with that from virgin monomer. This is in particular challenging, since the produced polymethyl methacrylate and thus the methyl methacrylate used as monomer needs to be very pure for most of the applications of polymethyl methacrylate, such as for instance for its use as dental prothesis or as medical prothesis, but also for non-medical applications, such as for example optical grade polymethyl methacrylate for its use as plastic cover of an automotive rear and/or front light, for which very transparent polymethyl methacrylate, i.e. one with virtually no impurities and color, is required. On account thereof, a recycling of polymethyl methacrylate via pyrolysis of polymethyl methacrylate to methyl methacrylate necessitates a nearly complete removal of the impurities from the pyrolyzed composition, such as of esters and diesters of carboxylic acids, alcohols, carboxylic acids, ketones and others. However, the known purification methods based on several distillation steps are energy intensive and connected with high operational costs and high capital expenditures.

In view of this, the object underlying the present invention is to provide a method for preparing a purified methyl methacrylate composition from a crude composition containing methyl methacrylate, such as in particular from a crude composition being prepared by pyrolysis of a composition containing recycled polymethyl methacrylate, which leads to very pure polymethyl methacrylate, which contains, if at all, only minimal, non-disturbing amounts of impurities, wherein the method is nevertheless characterized by low operational and capital expenditures.

In accordance with the present invention, this object is satisfied by providing a method for preparing a purified methyl methacrylate composition from a crude composition containing methyl methacrylate comprising the steps of:
a) providing as crude composition a pyrolyzed composition obtained by pyrolysis of a polymethyl methacrylate composition,
b) subjecting the pyrolyzed composition to at least one distillation step so as to obtain a methyl methacrylate enriched composition and
c) then subjecting the methyl methacrylate enriched composition to at least one melt crystallization step so as to obtain the purified methyl methacrylate composition.

This solution bases on the finding that by subjecting a crude composition containing methyl methacrylate, such as in particular a crude composition being prepared by pyrolysis of a composition containing recycled polymethyl methacrylate, to at least one distillation step followed by at least one melt crystallization step, a very pure methyl methacrylate composition with a methyl methacrylate content of 99.8% by weight or more and a total impurity content of as low as 50 ppm or less is obtained. In particular, the at least one melt crystallization step allows to efficiently remove impurities having a boiling point being close to that of methyl methacrylate, which are, if at all, only removable by distillation with very high energy costs. On account of the high purity of the resulting purified methyl methacrylate, the produced purified methyl methacrylate is particularly suitable to be used as monomer for polymethyl methacrylate compositions used for preparing dental protheses, medical protheses, plastic covers of automotive rear and front lights or other applications, in which very pure polymethyl methacrylate is required. Another advantage of the method in accordance with the present invention is that it has a high yield, namely a yield of more than 90% by weight of methyl methacrylate being contained in the crude composition or pyrolyzed composition, respectively. Furthermore, due to the use of crystallization, the total operational costs of the method as well as the capital expenditures are comparable low. All in all, the method in accordance with the present invention does not only lead to very pure methyl methacrylate, but is also characterized by low operational and capital expenditures.

The present invention is not particularly limited concerning the kind of crude composition being provided in step a), as long as the crude composition is a pyrolyzed composition being obtained by pyrolysis of a polymethyl methacrylate composition and preferably a recycled polymethyl methacrylate composition. Thus, preferably the method comprises as step a) a step of subjecting a polymethyl methacrylate composition to at least one pyrolysis step so as to obtain the pyrolyzed composition. The (recycled) polymethyl methacrylate composition used for the pyrolysis may be a virgin polymethyl methacrylate composition, such as production waste or the like. Preferably, the polymethyl methacrylate composition used for the pyrolysis is a post-consumer polymethyl methacrylate composition. Before being subjected to the pyrolysis, the recycled polymethyl methacrylate may be subjected to one or more preparation steps, such as an automatic sorting, one or more shredding steps and/or one or more separation steps, in which non-plastic materials are separated from the plastic material(s).

For instance, the (recycled) polymethyl methacrylate composition used in step a) contains at least 80% by weight of polymethyl methacrylate from mixed scraps. Good results are in particular obtained, when the (recycled) polymethyl methacrylate composition used in step a) contains at least 90% by weight and most preferably 90 to 100% by weight of polymethyl methacrylate.

Preferably, the polymethyl methacrylate composition is molten to a melt, before the melt is pyrolyzed in step a). The present invention is not particularly limited concerning the kind, how the polymethyl methacrylate composition is molten to a melt. Good results are in particular obtained, when the polymethyl methacrylate composition is molten in an extruder, such as in a single screw extruder or in a twin-screw extruder. An extruder does not only pre-heat and melt the polymethyl methacrylate composition, but also mixes it in a continuous manner. Good results are in particular obtained, when the polymethyl methacrylate composition is heated in the extruder to a temperature of 190 to 250°C and preferably to 210 to 240°C. The polymethyl methacrylate composition may be fed into the extruder in any form, such as in the form of chips, flakes, pellets, powder or the like.

In accordance with the present invention, the (melt of) the polymethyl methacrylate composition is then subjected to at least one pyrolysis step so as to obtain the pyrolyzed composition as crude composition. Good results are in particular obtained, when the at least one pyrolysis step is performed at a temperature of 400 to 600°C and more preferably at a temperature of 450 to 520°C. Thereby, the polymethyl methacrylate is decomposed with high yield into methyl methacrylate.

In a further development of the idea of the present invention, it is proposed that the at least one pyrolysis step is performed in an isothermal reactor or in an adiabatic reactor. Good results are in particular obtained, when a fluidized bed is used as isothermal reactor.

The pyrolysis is preferably performed in an inert gas atmosphere, such as in nitrogen or a noble gas, or in the atmosphere of the formed product.

The pyrolysis products or pyrolyzed composition, respectively, preferably contains more than 80% by weight, more preferably more than 90% by weight and most preferably more than 95% by weight of methyl methacrylate, wherein the rest to 100% by weight are impurities. The impurities comprise methyl esters of carboxylic acids, ethyl and butyl esters of carboxylic acids, hydrocarbons, alcohols, diesters of carboxylic acids, ketones and carboxylic acids. Examples for methyl esters of carboxylic acids are methyl esters of acrylic acid, propionic acid, isobutyric acid and benzoic acid, whereas examples of ethyl and butyl esters of carboxylic acids are those of acrylic acid. In turn, the hydrocarbon impurities are pentene, 2-pentene, cyclopentene, toluene, benzene, propyl benzene, ethyl benzene and/or the like, whereas the alcohol impurities are mainly methanol and ethanol, whereas an example for a diester of carboxylic acids being contained in the pyrolyzed composition is ethylene glycol dimethacrylate, examples for phthalates are phthalic anhydride and dibutylphthalate, an example for a ketone is dimethyl cyclopente-none and an example for a carboxylic acid is methacrylic acid. During the pyrolysis reaction by-products are formed, such as methylisobutyrate, ethylacrylate, methylacrylate, methylproprionate, methanol, water and others having a boiling point being close to that of methyl methacrylate or which form an azeotrope with methyl methacrylate. These by-products are removed from the pyrolyzed composition in accordance with the present invention mainly during the at least one melt crystallization step.

In accordance with one embodiment of the present invention, step b) may comprise (only) one distillation step. In this embodiment, the pyrolyzed composition is fed in step b) into a distillation column and is distilled therein into an overhead fraction, into a bottom fraction and into a side fraction, wherein the side fraction is led as the (pre-purified, namely) methyl methacrylate enriched composition to the at least one melt crystallization step c). Preferably, the temperature inside the distillation column is adjusted in step b) to be lower than 95°C and more preferably to be lower than 80°C, whereas the distillation columns are connected to a vacuum system, wherein the pressure inside the distillation columns is preferably adjusted in step b) to 5 to 75 kPa.

In a further development of the idea of the present invention, it is suggested that the pyrolyzed composition is subjected in step b) to two or more subsequent distillation steps and more preferably to two subsequent distillation steps. In this embodiment, the pyrolyzed composition is fed in step b) into a first distillation column and is distilled therein into an overhead fraction and into a bottom fraction, wherein the bottom fraction is led into a second distillation column and is distilled therein into an overhead fraction and into a bottom fraction. While the overhead fraction obtained in the first distillation column contains lights, such as methanol and ethanol, and the bottom fraction obtained in the second distillation column contains heavies, such as phthalates and diesters, the overhead fraction obtained in the second distillation column is led as methyl methacrylate enriched composition into the at least one melt crystallization step.

Also in this embodiment it is preferred that the temperature in each of the distillation column is adjusted in step b) to lower than 95°C and more preferably to lower than 80°C, whereas each of the distillation columns is connected to a vacuum system, wherein the pressure in each of the distillation columns is preferably adjusted in step b) to 5 to 75 kPa.

The methyl methacrylate enriched composition obtained in this embodiment contains preferably more than 98% by weight, more preferably at least 99% by weight, yet more preferably at least 99.5% by weight and most preferably 99.5% to 99.8% by weight of methyl methacrylate.

In accordance with an alternative, particularly preferred embodiment of the present invention, the pyrolyzed composition provided in step a) is subjected in step b) to a distillation step in a dividing-wall distillation column. This allows to obtain a (pre-purified, namely) methyl methacrylate enriched composition with low operational costs and with low capital expenditures. Concerning the kind of dividing-wall distillation column, the present invention is not particularly restricted. Thus, any distillation column may be used, which comprises a dividing wall, which separates at least a longitudinally extending section of the dividing distillation column, seen in its cross-section, into two sub-sections. Preferably, the dividing wall is arranged in the dividing-wall distillation column at least essentially vertical downwards. At least essentially vertically downwards means in accordance with the present invention that the angle between the dividing wall and the length axis of the dividing-wall distillation column or vertical direction, respectively, is at most 20°, preferably at most 10°, more preferably at most 5° and most preferably 0°.

In accordance with a particularly preferred variant of this embodiment of the present invention, the pyrolyzed composition is fed in step b) into a middle dividing-wall distillation column and is distilled therein into an overhead composition, into a side composition as methyl methacrylate enriched composition and into a bottom composition, wherein the side fraction is led as methyl methacrylate enriched composition into the at least one melt crystallization step c).

A middle dividing-wall distillation column is defined according to the present invention as a dividing-wall distillation column, which comprises a dividing wall extending from a point being located below the top of the distillation column at least essentially vertically downwards to a point being located above the bottom of the dividing-wall distillation column so as to subdivide the dividing-wall distillation column into a top section being located above the dividing-wall, into a bottom section being located below the dividing-wall and into a middle section comprising a first middle subsection being located on one side of the dividing wall and a second middle subsection being located on the opposite side of the dividing wall. Good results are in particular obtained, when the dividing wall extends, seen from the bottom to the top of the dividing-wall distillation column, from a point being located at 10 to 45% of the distance from the bottom to the top of the dividing-wall distillation column to a point being located at 50 to 90% of the distance from the bottom to the top of the dividing-wall distillation column and preferably from a point being located at 25 to 40% of the distance from the bottom to the top of the dividing-wall distillation column to a point being located at 60 to 80% of the distance from the bottom to the top of the dividing-wall distillation column. Moreover, it is preferred that the dividing wall extends, seen from the bottom to the top of the dividing-wall distillation column, over 5 to 90%, more preferably over 20 to 80% and most preferably over 30 to 70% of the distance from the bottom to the top of the dividing-wall distillation column.

In a further development of the idea of the present invention, it is suggested that the dividing wall subdivides the middle section of the dividing-wall distillation column, seen in cross-section of the dividing-wall distillation column, in about two equally sized subsections or halves, respectively. Therefore, it is preferred that one of the first and second middle subsections covers at least 30%, more preferably at least 40%, yet more preferably at least 45% and most preferably 50% of the total area of the cross-section of the middle section of the dividing-wall column, whereas the other of the first and second middle subsections covers the remainder to 100% of the total area of the cross-section of the middle section of the dividing-wall column. For instance, one subsection covers 43% and the other 57% of the total area of the cross-section or both subsections cover each exactly 50% of the total area of the cross-section. If the total area of the cross-section of the middle section varies over the axial length of the middle section, the above numeric values are related to the average total area of the cross-section of the middle section.

Preferably, the distillation is performed in the middle dividing-wall distillation column at a temperature of below 95°C and at a pressure of below 75 kPa and more preferably at a temperature of below 80°C and at a pressure of below 55 kPa.

The side stream being obtained in the dividing-wall distillation column may or may not be subjected to a second distillation step. Preferably, the side stream being obtained in the dividing-wall distillation column is not subjected to a second distillation step, but directly led into the at least one melt crystallization step.

Also the methyl methacrylate enriched composition obtained in this embodiment contains preferably more than 98% by weight, more preferably at least 99% by weight, yet more preferably at least 99.5% by weight and most preferably 99.5% to 99.8% by weight of methyl methacrylate.

In order to further increase the purity degree of the methyl methacrylate by removing by-products being produced during the pyrolysis which have a boiling point being close to that of methyl methacrylate or which form an azeotrope with methyl methacrylate, such as methylisobutyrate, ethylacrylate, methylacrylate, methylproprionate, methanol, water and others, from the methyl methacrylate, the methyl methacrylate enriched composition obtained in the at least distillation step b) is according to the present invention subjected to at least one melt crystallization step c). Good results are in particular obtained, when the methyl methacrylate enriched composition is subjected in step c) to one to ten, more preferably to one to three and most preferably to one or two melt crystallization steps so as to obtain the purified methyl methacrylate composition.

The present invention is not particularly limited concerning the kind of the at least one melt crystallization step. Preferably, the at least one melt crystallization step b) comprises at least one melt crystallization step being selected from the group consisting of static crystallization steps, suspension crystallization steps and falling film crystallization steps.

Good results are in particular obtained, when the at least one melt crystallization step b) is a static crystallization step. Static crystallization is typically performed in a crystallizer or vessel, respectively, in which vertical plates being heated or cooled by an internal circulation of heat transfer medium are arranged. The melt to be crystallized, i.e. the methyl methacrylate enriched composition, is filled in the void between the vertical plates so that in fact the vertical plates dip into the methyl methacrylate enriched composition. By slowly cooling the heat transfer medium below the freezing point of methyl methacrylate, the formation of a layer of methyl methacrylate crystals on the surfaces of the vertical plates is initiated.

For instance, the pyrolyzed composition being subjected in step c) to one to ten static melt crystallization steps is fed into a first of the two to ten static melt crystallization steps so as to produce a first methyl methacrylate enriched crystallized fraction and a methyl methacrylate depleted residue fraction, wherein the first methyl methacrylate enriched crystallized fraction is fed into a second of the two to ten static melt crystallization steps, wherein in any of the second and of the optional third to ten static melt crystallization steps a methyl methacrylate enriched crystallized fraction and a methyl methacrylate depleted residue fraction is produced, wherein each of the methyl methacrylate enriched crystallized fractions produced in the second and the optional third to tenth static melt crystallization steps is fed into a downstream crystallization step and each of the methyl methacrylate depleted residue fractions produced in the second and the optional third to tenth static melt crystallization steps is fed into an upstream static melt crystallization step, wherein the methyl methacrylate enriched crystallized fraction of the most downstream of the static melt crystallization steps is the purified methyl methacrylate composition.

For example, the production of a methyl methacrylate enriched crystallized fraction and a methyl methacrylate depleted residue fraction in the static melt crystallization step comprises the sub-steps of removing the remaining liquid from the crystallization step as methyl methacrylate depleted residue fraction after termination of the crystallization in the static melt crystallization step, of melting the crystal layer obtained in the static melt crystallization step and of withdrawing the obtained crystal melt as methyl methacrylate enriched crystallized fraction from the crystallization step.

Good results are in particular obtained, when at least one and more preferably all of the at least one melt crystallization steps are performed at a crystallization temperature of -10°C to -80°C, preferably at a crystallization temperature of -25°C to - 70°C and more preferably at a crystallization temperature of -35°C to -60°C.

In order to increase the purity of the purified methyl methacrylate composition obtained in the at least one melt crystallization step, it is preferred that at least one sweating sub-step is performed in the at least one melt crystallization step. Sweating means that the crystal layer deposited on the cooled surfaces are gently heated to a temperature close to the melting temperature of methyl methacrylate in order to partially melt the crystals. Trapped and adherent melt, which contains the impurities, drains off during the partial melting of the crystals and is then removed from the crystallizer. In order to conduct such a sweating, the surface, on which the crystals are deposited, is heated with a heat transfer medium to the desired temperature. The sweating may be performed for one or several times before melting the crystal layers deposited on the cooled surfaces. Thus, the sweating leads to one or more sweating fractions and to a purified crystal layer. Preferably, at least a portion of the first sweating fraction obtained thereby is fed to the remaining liquid which has been removed as methyl methacrylate depleted residue fraction.

In accordance with still another preferred embodiment of the present invention, the purified methyl methacrylate composition obtained in the at least one melt crystallization step comprises at least 99.8% by weight of methyl methacrylate and 1,000 ppm or less, preferably 600 ppm or less, more preferably 250 ppm or less, still more preferably 50 ppm or less and most preferably 10 ppm or less impurities. It is in particular preferred, when the purified methyl methacrylate composition obtained in the at least one melt crystallization step has a APHA color of at most 5, wherein APHA color is a color standard named for the American Public Health Association as defined by ASTM D1209.

In accordance with another aspect, the present invention relates to a plant for preparing a purified methyl methacrylate composition from a (recycled) polymethyl methacrylate composition, wherein the plant comprises:
a) at least one pyrolysis reactor comprising an inlet for the polymethyl methacrylate composition and an outlet for pyrolyzed composition,
b) at least one distillation column comprising an inlet being connected with the outlet for the pyrolyzed composition of the at least one pyrolysis reactor, an overhead outlet and a bottom outlet and
c) at least one melt crystallizer comprising an inlet being connected with one of the outlets of the at least one distillation column as well as an outlet for purified methyl methacrylate composition.

Preferably, the plant further comprises an extruder, which is arranged upstream of the at least one pyrolysis reactor, wherein the extruder comprises an inlet for the polymethyl methacrylate composition and an outlet for melt of polymethyl methacrylate composition, wherein the outlet for melt of the extruder is connected with the inlet of the at least one pyrolysis reactor. The extruder may be for instance a single screw extruder or a twin-screw extruder.

In accordance with a preferred embodiment of the present invention, the plant comprises two distillation columns, wherein the first distillation column comprises an inlet being connected with the outlet for pyrolyzed composition of the at least one pyrolysis reactor, an overhead outlet and a bottom outlet, and wherein the second distillation column comprises an inlet being connected with the bottom outlet of the first distillation column, an overhead outlet for the methyl methacrylate enriched composition and a bottom outlet.

In accordance with an alternative, particularly preferred embodiment of the present invention, the plant comprises a dividing-wall distillation column, which comprises an inlet being connected with the outlet for pyrolyzed composition of the at least one pyrolysis reactor, an outlet for an overhead composition, an outlet for a side composition being the methyl methacrylate enriched composition and an outlet for a bottom composition.

In a further development of the idea of the present invention, it is suggested that the dividing-wall distillation column is a middle dividing-wall distillation column, which comprises a dividing wall extending from a point being located below the top of the distillation column at least essentially vertically downwards to a point being located above the bottom of the dividing-wall distillation column so as to subdivide the dividing-wall distillation column into a top section being located above the dividing-wall, into a bottom section being located below the dividing-wall and into a middle section comprising a first middle subsection being located on one side of the dividing wall and a second middle subsection being located on the opposite side of the dividing wall.

Good results are in particular obtained, when the dividing wall extends, seen from the bottom to the top of the dividing-wall distillation column, from a point being located at 10 to 45% of the distance from the bottom to the top of the dividing-wall distillation column to a point being located at 50 to 90% of the distance from the bottom to the top of the dividing-wall distillation column and preferably from a point being located at 25 to 40% of the distance from the bottom to the top of the dividing-wall distillation column to a point being located at 60 to 80% of the distance from the bottom to the top of the dividing-wall distillation column. Moreover, it is preferred that the dividing wall extends, seen from the bottom to the top of the dividing-wall distillation column, over 5 to 90%, more preferably over 20 to 80% and most preferably over 30 to 70% of the distance from the bottom to the top of the dividing-wall distillation column.

In a further development of the idea of the present invention, it is suggested that the dividing wall subdivides the middle section of the dividing-wall distillation column, seen in cross-section of the dividing-wall distillation column, in about two equally sized subsections or halves, respectively. Therefore, it is preferred that one of the first and second middle subsections covers at least 30%, more preferably at least 40%, yet more preferably at least 45% and most preferably 50% of the total area of the cross-section of the middle section of the dividing-wall column, whereas the other of the first and second middle subsections covers the remainder to 100% of the total area of the cross-section of the middle section of the dividing-wall column. For instance, one subsection covers 43% and the other 57% of the total area of the cross-section or both subsections cover each exactly 50% of the total area of the cross-section. If the total area of the cross-section of the middle section varies over the axial length of the middle section, the above numeric values are related to the average total area of the cross-section of the middle section.

In accordance with a further particularly preferred embodiment of the present invention, the plant comprises as at least one melt crystallizer c) at least one static melt crystallizer.

In the embodiment, in which the plant comprises at least one distillation column, it is preferred that the plant comprises one to three and more preferably one or two static melt crystallizers.

The invention will be explained in more detail hereinafter with reference to the drawings, in which:

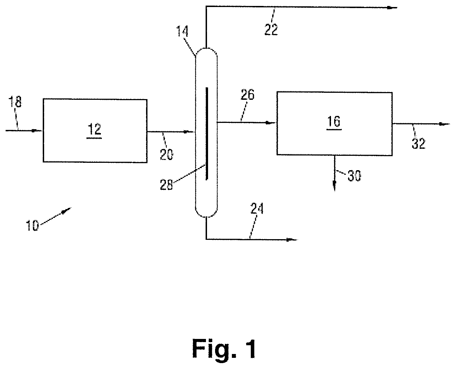

- Fig. 1: schematically shows a plant for preparing a purified methyl methacrylate composition from a recycled polymethyl methacrylate composition in accordance with one embodiment of the present invention.

The plant 10 for preparing a purified methyl methacrylate composition from a recycled polymethyl methacrylate composition shown in figure 1 comprises a pyrolysis reactor 12, a distillation column 14 and a static melt crystallizer 16. More specifically, the pyrolysis reactor 12 comprises an inlet line 18 for feed, such as a melt of recycled polymethyl methacrylate composition. For preparing the melt, the plant 10 may further comprise upstream of the pyrolysis reactor 12 an extruder (not shown). In addition, the pyrolysis reactor 12 comprises an outlet line 20 for a pyrolyzed liquid composition or liquid crude composition, respectively, which is simultaneously the inlet line 20 for pyrolyzed composition of the distillation column 14. The distillation column 14 is a middle divided-wall distillation column 14, which comprises a dividing wall 28, which extends, seen over the height of the middle divided-wall distillation column 14, which is the straight distance between the bottom and the top of the middle divided-wall column 14, vertically downwards from about 25% to about 75% of the height of the middle divided-wall column 14. The middle divided-wall column 14 further comprises an overhead outlet line 22 for lights, such as ethanol, a bottom outlet line 24 for heavies, such as phthalates and diesters, and a side outlet line 26 for a methyl methacrylate enriched composition. The side outlet line 26 for a methyl methacrylate enriched composition of the middle divided-wall column 14 is the inlet line 26 of the static melt crystallizer 16. In turn, the static melt crystallizer 16 comprises a discharge conduit 30 for a methyl methacrylate depleted residue fraction and a discharge conduit 32 for a purified methyl methacrylate composition.

During the operation, a melt of recycled polymethyl methacrylate, which has been for instance produced in a single screw extruder, is fed via line 18 into the pyrolysis reactor 12, in which the polymethyl methacrylate is thermally decomposed to methyl methacrylate and side-products. The pyrolyzed composition, which contains methyl methacrylate and the side-products as impurities, such as esters of carboxylic acids, alcohols, diesters, phthalates, ketones, carboxylic acids and other hydrocarbons, is fed via the line 20 as crude composition into the middle divided-wall column 14. In the middle divided-wall column 14, the crude composition is separated into a C₄-hydrocarbon stream obtained as overhead fraction, which mainly contains ethanol, into a methyl methacrylate enriched composition obtained as side fraction and into a C₆₊-hydrocarbon stream obtained as bottom fraction, which contains for instance phthalates and diesters. While the overhead fraction is withdrawn from the middle divided-wall column 14 via line 22 and the bottom fraction is withdrawn from the middle divided-wall column 14 via line 24, the side fraction or methyl methacrylate enriched composition, respectively, is withdrawn from the middle divided-wall column 14 via line 26 and fed via line 26 into the static melt crystallizer 26. The methyl methacrylate enriched composition is further purified in the static melt crystallizer 16 by separating by-products, such as methylisobutyrate, ethylacrylate, methylacrylate, methylproprionate, methanol, water and others being produced during the pyrolysis which have a boiling point being close to that of methyl methacrylate or which form an azeotrope with methyl methacrylate, from the methyl methacrylate, wherein the so obtained methyl methacrylate depleted residue fraction is withdrawn from the static melt crystallizer 16 via the discharge conduit 30 and the so obtained purified methyl methacrylate composition is withdrawn from the static melt crystallizer 16 via the discharge conduit 32.

### Reference Numerals

- 10: Plant for preparing a purified methyl methacrylate composition
- 12: Pyrolysis reactor
- 14: (Middle) divided-wall distillation column
- 16: Static melt crystallizer
- 18: Inlet line for feed
- 20: Outlet line for pyrolyzed composition / inlet line for pyrolyzed composition
- 22: Overhead outlet line of the distillation column
- 24: Bottom outlet line of the distillation column
- 26: Side outlet line of the distillation column / inlet line of the static melt crystallizer
- 28: Dividing wall of the distillation column
- 30: Discharge conduit for a methyl methacrylate depleted residue fraction
- 32: Discharge conduit for a purified methyl methacrylate composition

## Claims

1. A method for preparing a purified methyl methacrylate composition from a crude composition containing methyl methacrylate comprising the steps of:
a) providing as crude composition a pyrolyzed composition obtained by pyrolysis of a polymethyl methacrylate composition,
b) subjecting the pyrolyzed composition to at least one distillation step so as to obtain a methyl methacrylate enriched composition and
c) then subjecting the methyl methacrylate enriched composition to at least one melt crystallization step so as to obtain the purified methyl methacrylate composition.

2. The method in accordance with claim 1, wherein step a) comprises a step of subjecting a polymethyl methacrylate composition to at least one pyrolysis step so as to obtain the pyrolyzed composition

3. The method in accordance with claim 1 or 2, wherein the polymethyl methacrylate composition used in step a) contains at least 80% by weight, preferably weight at least 90% by weight and most preferably 90 to 100% by weight of polymethyl methacrylate.

4. The method in accordance with any of the preceding claims, wherein step b) comprises two distillation steps, wherein the pyrolyzed composition is fed into a first distillation column and is distilled therein into an overhead fraction and into a bottom fraction, wherein the bottom fraction is led into a second distillation column and is distilled therein into an overhead fraction and into a bottom fraction, wherein the overhead fraction is led as methyl methacrylate enriched composition into the at least one melt crystallization step c).

5. The method in accordance with any of claims 1 to 3, wherein step b) comprises one distillation step in a dividing-wall distillation column and preferably in a middle dividing-wall distillation column, wherein the pyrolyzed composition is distilled therein into an overhead composition, into a side composition and into a bottom composition, wherein the side fraction is led as methyl methacrylate enriched composition into the at least one melt crystallization step.

6. The method in accordance with claim 5, wherein the middle dividing-wall distillation column comprises a dividing wall which extends, seen from the bottom to the top of the middle dividing-wall distillation column, from a point being located at 10 to 45% of the distance from the bottom to the top of the dividing-wall distillation column to a point being located at 50 to 90% of the distance from the bottom to the top of the dividing-wall distillation column, wherein the dividing wall extends, seen from the bottom to the top of the dividing-wall distillation column, over 5 to 90%, more preferably over 20 to 80% and most preferably over 30 to 70% of the distance from the bottom to the top of the dividing-wall distillation column.

7. The method in accordance with any of the preceding claims, wherein i) the methyl methacrylate enriched composition is subjected in step b) to at least one, preferably one to ten, more preferably one to three and most preferably one or two static melt crystallization steps so as to obtain the purified methyl methacrylate composition.

8. The method in accordance with claim 7, wherein the pyrolyzed composition being subjected in step b) to one to ten static melt crystallization steps is fed into a first of the two to ten static melt crystallization steps so as to produce a first methyl methacrylate enriched crystallized fraction and a methyl methacrylate depleted residue fraction, wherein the first methyl methacrylate enriched crystallized fraction is fed into a second of the two to ten static melt crystallization steps, wherein in any of the second and of the optional third to ten static melt crystallization steps a methyl methacrylate enriched crystallized fraction and a methyl methacrylate depleted residue fraction is produced, wherein each of the methyl methacrylate enriched crystallized fractions produced in the second and the optional third to tenth static melt crystallization steps is fed into a downstream crystallization step and each of the methyl methacrylate depleted residue fractions produced in the second and the optional third to tenth static melt crystallization steps is fed into an upstream static melt crystallization step, wherein the methyl methacrylate enriched crystallized fraction of the most downstream of the static melt crystallization steps is the purified methyl methacrylate composition.

9. The method in accordance with claim 8, wherein producing a methyl methacrylate enriched crystallized fraction and a methyl methacrylate depleted residue fraction in a static melt crystallization step comprises the sub-steps of removing the remaining liquid from the crystallization step as methyl methacrylate depleted residue fraction after termination of the crystallization in the static melt crystallization step, of melting the crystal layer obtained in the static melt crystallization step and of withdrawing the obtained crystal melt as methyl methacrylate enriched crystallized fraction from the crystallization step, wherein preferably before melting the crystal layer obtained in the crystallization step, one or more sweating steps of the crystal layer are carried out so as to obtain one or more sweating fractions and a purified crystal layer.

10. The method in accordance with any of the preceding claims, wherein at least one and preferably all of the at least one melt crystallization steps are performed at a crystallization temperature of -10°C to -80°C, preferably at a crystallization temperature of -25°C to -70°C and more preferably at a crystallization temperature of -35°C to -60°C.

11. The method in accordance with any of the preceding claims, wherein the purified methyl methacrylate composition comprises at least 99.8% by weight of methyl methacrylate and 1,000 ppm or less, preferably 600 ppm or less, more preferably 250 ppm or less, still more preferably 50 ppm or less and most preferably 10 ppm or less impurities.

12. A plant for preparing a purified methyl methacrylate composition from a polymethyl methacrylate composition, wherein the plant comprises:
a) at least one pyrolysis reactor comprising an inlet for the polymethyl methacrylate composition and an outlet for pyrolyzed composition,
b) at least one distillation column comprising an inlet being connected with the outlet for the pyrolyzed composition of the at least one pyrolysis reactor, an overhead outlet and a bottom outlet and
c) at least one melt crystallizer comprising an inlet being connected with one of the outlets of the at least one distillation column as well as an outlet for purified methyl methacrylate composition.

13. The plant in accordance with claim 12, which further comprises an extruder, preferably a single screw extruder or twin-screw extruder, which is arranged upstream of the at least one pyrolysis reactor, wherein the extruder comprises an inlet for the polymethyl methacrylate composition and an outlet for melt of polymethyl methacrylate composition, wherein the outlet for melt of the extruder is connected with the inlet of the at least one pyrolysis reactor.

14. The plant in accordance with claims 12 or 13, which i) comprises two distillation columns, wherein the first distillation column comprises an inlet being connected with the outlet for pyrolyzed composition of the at least one pyrolysis reactor, an overhead outlet and a bottom outlet, and wherein the second distillation column comprises an inlet being connected with the bottom outlet of the first distillation column, an overhead outlet for the methyl methacrylate enriched composition and a bottom outlet, or ii) which comprises a middle dividing-wall distillation column comprising an inlet being connected with the outlet for pyrolyzed composition of the at least one pyrolysis reactor, an outlet for an overhead composition, an outlet for a side composition of the methyl methacrylate enriched composition and an outlet for a bottom composition, wherein the middle dividing-wall distillation column further comprises a dividing wall extending from a point being located below the top of the distillation column at least essentially vertically downwards to a point being located above the bottom of the dividing-wall distillation column so as to subdivide the dividing-wall distillation column into a top section being located above the dividing-wall, into a bottom section being located below the dividing-wall and into a middle section comprising a first middle subsection being located on one side of the dividing wall and a second middle subsection being located on the opposite side of the dividing wall.

15. The plant in accordance with any of claims 12 to 14, wherein the plant comprises one to three and preferably one or two static melt crystallizers b).
